# FASCICULE DE BREVET EUROPEEN

(11) **EP 3 086 112 B1**
(45) Date de publication et mention de la délivrance du brevet: **31.01.2018**
(21) Numéro de dépôt: 16165876.0
(22) Date de dépôt: 18.04.2016
(51) Int. Cl.: G01N 27/12, G01N 33/487

(54) **DISPOSITIF ÉLECTRONIQUE DE MESURE D'AU MOINS UNE CARACTÉRISTIQUE ÉLECTRIQUE D'UN OBJET**
ELEKTRONISCHE VORRICHTUNG ZUM MESSEN MINDESTENS EINES ELEKTRISCHEN PARAMETERS EINES GEGENSTANDS
ELECTRONIC DEVICE FOR MEASURING AT LEAST ONE ELECTRICAL CHARACTERISTIC OF AN OBJECT

(30) Priorité: 20.04.2015 FR 1553533
(43) Date de publication de la demande: 26.10.2016
(73) Titulaire: COMMISSARIAT À L'ÉNERGIE ATOMIQUE ET AUX ÉNERGIES ALTERNATIVES, 75015 Paris (FR)
(72) Inventeur: CARMIGNANI, Corentin, 38000 GRENOBLE (FR); BRUN, Christophe, 38000 GRENOBLE (FR); REYNAUD, Patrick, 38420 MURIANETTE (FR); ROLLAND, Emmanuel, 38560 JARRIE (FR)
(74) Mandataire: Decobert, Jean-Pascal

(56) Documents cités:
- EP-A1- 2 833 129
- EP-A2- 2 333 517
- WO-A1-2013/039468
- DE-A1-102011 077 875
- DE-U1- 29 501 233
- US-A1- 2011 259 079
- US-A1- 2013 292 266
- WENBIN XUE ET AL: "New Four-Band Electrode Fabrication To Measure in Situ Electrical Property of Conducting Polymers", ANALYTICAL CHEMISTRY, vol. 81, no. 6, 15 mars 2009 (2009-03-15), pages 2364-2372, XP055283425, ISSN: 0003-2700, DOI: 10.1021/ac801799h
- PAUL E ET AL: "Resistance of Polyaniline Films as a Function of Electrochemical Potential and the Fabrication of Polyaniline-Based Microwlwctronic Devices", JOURNAL OF PHYSICAL CHEMISTRY, AMERICAN CHEMICAL SOCIETY, US, vol. 89, 1 janvier 1985 (1985-01-01), pages 1441-1447, XP007915224, ISSN: 0022-3654

## Description

### DOMAINE DE L'INVENTION

L'invention est relative à un dispositif et à un procédé de mesure d'au moins une caractéristique électrique d'un objet.

Elle s'adresse préférentiellement à la caractérisation d'objets de dimensions réduites, micrométriques voire nanométriques. Ces objets peuvent être notamment en partie constitués ou obtenus par de la matière biologique, en particulier de l'ADN, de protéines, des molécules organiques, des anticorps de l'ADN métallisé, des nanotubes....

Une caractérisation possible est la détermination de la contribution de l'objet au passage d'un courant au travers d'un circuit fermé par ledit objet. On peut ainsi étudier le transport électrique de l'objet.

### ÉTAT DE LA TECHNIQUE

Il existe des dispositifs de caractérisation d'objets employant des électrodes utilisées pour servir de zones de contact avec l'objet pour la conduction électrique.

Dans ce cadre, la publication d'Elizabeth W. PAUL et al parue dans « The Journal of Physical Chemistry », vol. 89, N°8 en 1985, intitulée « Resistance of Polyaniline films as a fonction of electrochemical potential and the fabrication of Polyaniline-based microelectronic devices » décrit une structure dotée d'une série d'électrodes parallèles, de même taille et indépendantes au niveau de la surface supérieure desquelles un objet en polyaniline peut être déposé. La grandeur du courant appliqué à un potentiel donné grâce aux électrodes dépend du potentiel électrochimique de la polyaniline et permet de caractériser l'objet en ce matériau. La mesure est simplement une mesure de courant entre deux électrodes.

La précision de mesure offerte par cette structure antérieure est limitée. En particulier, elle ne tient pas compte des phénomènes électriques parasites qui se produisent à l'interface entre les électrodes et l'objet à caractériser. Elle n'apporte pas non plus la possibilité d'effectuer une mesure statistique du courant conduit par un ou plusieurs nano objets. De plus, le positionnement d'objets est difficile sur de telles structures. Il en va de même dans la publication "New Four-Band Electrode Fabrication to Measure in Situ Electrical Property of Conducting Polymers" de Wenbin Xue et al publiée dans "Analytical Chemistry", vol. 81, N°6 en 2009.

EP2333517 A2, US2011259079 A1 et DE102011077875 A1 décrivent d'autres dispositifs comprenant au moins deux unités de mesure avec des électrodes interdigitées.

La présente invention permet de remédier en tout ou partie aux inconvénients techniques actuellement connus.

### RÉSUMÉ DE l'INVENTION

Un aspect de modes de réalisation de l'invention concerne un dispositif électronique de mesure d'au moins une caractéristique électrique d'un objet selon la revendication 1, comportant, entre autres, un support pourvu d'au moins deux unités de mesure comportant chacune au moins deux jeux d'électrodes avec i entier supérieure ou égal 2 , chaque jeu d'électrodes comprenant un nombre mi d'électrodes supérieur ou égal à un, les électrodes d'un jeu d'électrodes ayant une dimension en largeur, les électrodes des jeux d'électrodes d'une même unité de mesure étant interdigitées de sorte que chaque électrode de l'un des jeux d'électrodes Ji de ladite unité de mesure soit espacée d'une distance inter-électrodes d'une électrode, de l'autre des jeux d'électrodes Ji+1 de ladite unité de mesure, qui lui est adjacente; les électrodes des jeux d'électrodes d'une unité de mesure formant pour ladite unité de mesure, une zone d'accueil d'au moins un objet, chaque objet dans cette zone d'accueil définissant une zone de mise en contact discontinue avec les électrodes de l'unité de mesures, ladite zone de mise en contact ayant une longueur déterminée suivant la dimension en largeur (W) des électrodes correspondant à la somme des largeurs des électrodes en contact avec cet objet, et chaque zone d'accueil comportant en outre une zone inter-électrodes discontinue ayant une longueur prédéterminée suivant la dimension en largeur (W) des électrodes de ladite unité de mesure; un circuit de mesures raccordé aux jeux d'électrodes de chaque unité de mesure configuré pour déterminer le courant traversant l'objet; dispositif dans lequel au moins deux unités de mesure diffèrent entre elles par la longueur de leurs zones de mise en contact et/ou la longueur de leurs zones inter-électrodes.

Un autre aspect de modes de réalisation de l'invention s'adresse à un procédé de mesure d'au moins une caractéristique électrique d'un objet selon la revendication 14, comprenant :
- l'utilisation du dispositif selon l'invention;
- une étape de placement d'un objet sur la zone de mise en contact d'une unité de mesure et une étape de mesure du courant circulant entre les jeux d'électrodes de ladite unité;
- une étape de placement de l'objet ou d'un objet identique sur la zone de mise en contact d'une autre unité de mesure et une étape de mesure du courant circulant entre les jeux d'électrodes de ladite autre unité;
- une évaluation du courant traversant l'objet.

Un intérêt potentiel de l'invention est de permettre une mesure différentielle des courants passant au travers d'objets via les électrodes de sorte à maîtriser, voire annuler, les effets des phénomènes parasites tels que la formation de courants parasites au niveau des contacts entre l'objet et les électrodes. Ces courants parasites, dus aux résistances de contact, sont pris en compte de manière différentielle en employant plusieurs unités de mesure différant par la résistance de contact qu'elles occasionnent.

Un autre aspect de l'invention est un procédé de fabrication d'un dispositif de mesure.

Un autre aspect de l'invention est de pouvoir réaliser une mesure statistique de nano objets. Un grand nombre d'unités seront de préférence fabriquées sur un substrat et une étude statistique avec un grand nombre d'informations (W, G etc..) qui pourront être analysées

Un autre aspect de l'invention est de favoriser la déposition de petits outils tels des protéines. Il est difficile de contrôler le positionnement exact, notamment par dépôt d'un liquide contenant les nano objets entre deux électrodes. L'invention est donc particulièrement intéressante car du fait d'un grand nombre de dispositifs potentiellement fabriqués sur un substrat, on augmente la probabilité qu'au moins une protéine soit positionnée au mieux sur les électrodes.

L'invention propose donc un aspect industriel par l'utilisation de ces électrodes sur un substrat (exemples : silicium avec des plaques 100 mm, 200 mm ou 300 mm) permettant d'obtenir un grand nombre de nano objets sur un substrat conventionnel et utilisable par l'industrie.

Un autre aspect de l'invention est un système comprenant un dispositif selon l'invention et au moins un objet d'un type donné.

### INTRODUCTION DES DESSINS

Les dessins ci-joints sont donnés à titre d'exemples et ne sont pas limitatifs de l'invention. Ils représentent seulement un mode de réalisation de l'invention et permettront de la comprendre aisément.
- La figure 1 est une vue de dessus d'une face d'un support équipée de deux unités de mesure de l'invention dans un mode de réalisation de l'invention.
- La figure 2 est une vue de dessus d'une face d'un support équipée d'unités de mesure de l'invention dans un autre mode de réalisation.
- La figure 3 est une vue de dessus d'une face d'un support intégrant une pluralité d'unités de mesure de différentes conceptions.
- La figure 4 est une vue au microscope illustrant des électrodes interdigitées de deux d'électrodes.
- Les figures 5a et 5k schématisent des étapes successives d'une possibilité de procédé de fabrication d'un dispositif. Les figures 5l à 5m montrent deux variantes de résultat.
- La figure 6a montre schématiquement de dessus un exemple de réalisation d'électrodes pour une unité de mesure et la figure 6b en montre une vue en perspective coupée suivant la ligne AA de la figure 6a et schématise un circuit de mesure.

### DESCRIPTION DÉTAILLÉE

D'autres buts et avantages apparaîtront au cours de la description qui suit qui présente un mode de réalisation de l'invention illustratif mais non limitatif.

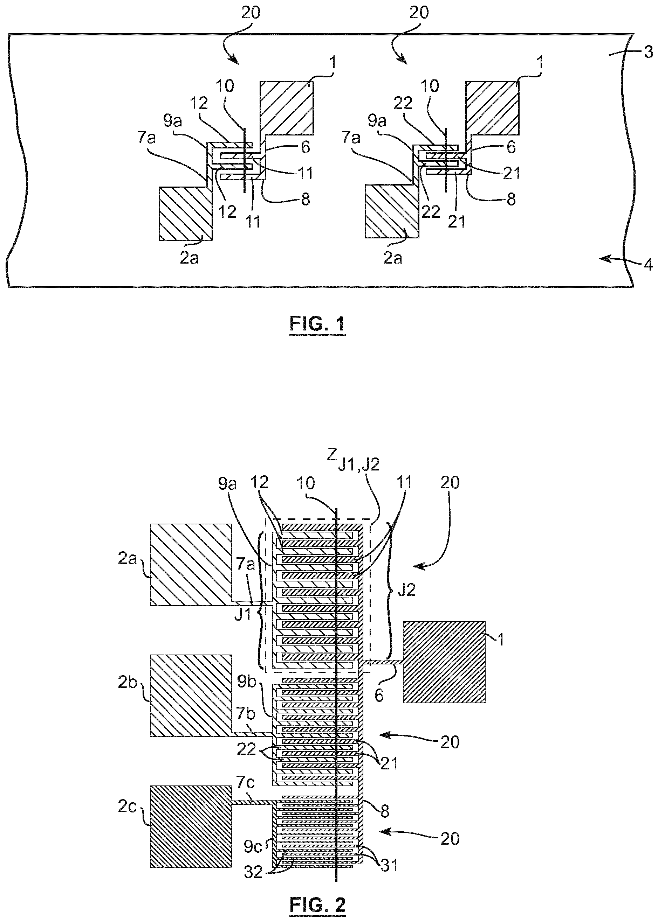

Suivant des variantes préférées mais non limitatives, l'invention est telle que :
- le nombre d'électrodes de chaque jeu d'une même unité de mesure peut être égal ou différent. Ainsi, bien que les figures représentent le même nombre d'électrodes pour chaque jeu, on pourrait avoir par exemple le jeu J1 (réf 2a fig. 2) avec un nombre m1 d'électrodes inférieur au nombre m2 d'électrodes du jeu J2 (réf 1 fig. 2) (par exemple m2=m1+1).
- ladite longueur de la zone de mise en contact correspond de préférence à la somme des largeurs (WJi) des parties des électrodes réellement en contact avec ledit objet. En particulier, les extrémités de l'objet ne recouvrant pas forcément toute la largeur d'une électrode, la largeur de celle-ci n'interviendra que partiellement.
- la zone de mise en contact peut être inférieure à la somme des largeurs de toutes les électrodes d'une unité de mesure lorsque l'objet ne recouvre pas toutes les électrodes de cette unité. Ainsi certaines des électrodes d'une unité peuvent ne pas entrer en compte dans cette zone de mise en contact ou que partiellement

- dans chaque jeu d'électrodes de chaque unité de mesure, la dimension en largeur des électrodes est identique.
- la longueur de la zone de mise en contact d'au moins deux unités de mesure diffère par la largeur des électrodes d'au moins un jeu d'électrodes de chaque desdites au moins deux unités de mesure.
- la longueur de la zone de mise en contact d'au moins deux unités de mesure diffère par le nombre (m) d'électrodes de leurs jeux d'électrodes.
- dans chaque jeu d'électrodes de chaque unité de mesure, la distance inter-électrodes est identique.
- les longueurs des zones inter-électrodes d'au moins deux unités de mesure diffèrent par leurs distances inter-électrodes;
- le circuit de mesure comporte des plots, les jeux d'électrodes d'une même unité de mesure étant reliés à des plots distincts ;
- le circuit de mesure comporte au moins un plot relié à plusieurs jeux d'électrodes appartenant chacune à une unité de mesure différente ;
- au moins un plot est situé sur une face du support différent d'une face sur laquelle est située l'unité de mesure à laquelle ledit au moins un plot est relié, et en ce que le circuit de mesure comprend une partie de raccordement, traversant avantageusement le support, entre ledit au moins un plot de l'unité de mesure;
- les électrodes des jeux d'électrodes de ladite au moins une unité de mesure sont affleurantes ;
- les électrodes des jeux d'électrodes de ladite au moins une unité de mesure sont espacées par des portions non conductrices affleurant sur la face du support ;
- la dimension en largeur (W) des électrodes est inférieure à 10 µm, et de préférence inférieure à 1 µm;
- la distance inter-électrodes est comprise entre 1 nm et 10 µm, et de préférence inférieure entre 500 nm et 1 µm;
- la longueur de la zone de mise en contact est inférieure à 1 nm ;
- les électrodes des jeux d'électrodes d'au moins une unité de mesure comportent une couche supérieure en Ruthénium;
- le support comporte une première face pourvue d'au moins une unité de mesure et une deuxième face différente de la première face, pourvue d'au moins une unité de mesure;
- le circuit de mesure comprend une alimentation électrique et une partie de mesure du courant circulant entre les jeux d'électrodes des unités de mesure en présence d'un objet sur la zone de contact des unités de mesure.

Les caractéristiques indiquées précédemment ne sont qu'optionnelles.

Un autre aspect de l'invention est un procédé de fabrication d'un dispositif de mesure tel qu'il comporte au moins une des options suivantes :
- on utilise un objet filiforme et on place l'objet sur la zone de mise en contact d'une unité de mesure de sorte qu'une dimension en longueur de l'objet soit dirigée suivant la dimension en largeur des électrodes de ladite unité de mesure;
- on utilise un objet ayant une dimension en longueur inférieure à 1 mm, et de préférence inférieure à 100 µm;
- on utilise un objet ayant une dimension en largeur inférieure à 10 µm.

D'une manière générale, l'invention permet, au moyen d'unités de mesure, d'opérer une mesure de caractéristiques électriques d'un objet, une mesure électrique pouvant être en particulier déduite de la contribution d'un courant passant au travers de l'objet lorsque ce dernier est placé de sorte à fermer un circuit entre deux jeux d'électrodes, le circuit comprenant alors une source électrique d'alimentation et des moyens de mesure de courant entre les électrodes.

L'invention permet notamment de caractériser les propriétés électriques et de conduction ou de contact d'objets pouvant servir à un grand nombre de dispositifs utilisés par exemple en tant que capteurs, interconnections d'objets. Les domaines d'applications de ces dispositifs étant par exemple la microélectronique, la médecine, la biologie, l'automobile, agro-alimentaire...

Un placement privilégié consiste à orienter l'objet transversalement à la dimension en largeur des électrodes. Cela est particulièrement préférable dans le cas d'objets filiformes, s'entendant ici d'objets ayant un rapport de forme élevée, par exemple supérieure à 10 voire 50. Ces objets peuvent par ailleurs être des micro ou des nano objets, c'est-à-dire qu'au moins une de leurs dimensions est à l'échelle, respectivement du micron ou du nanomètre. Par exemple, la longueur de l'objet peut être comprise entre 1Å et 1nm et plus préférentiellement entre 100nm et 100µm. La largeur ou le diamètre de l'objet peut par exemple être compris entre 1 nm et 10µm. L'objet peut être lui-même composé d'une ou plusieurs parties et donc être complexe tels des nano-objets bio inspirés issus de composants biologiques ou vivants. Plusieurs objets peuvent être mesurés simultanément.

L'aspect filiforme de l'objet n'est pas limitatif et l'invention s'applique notamment aussi à des objets sphériques.

Le ou les objets sont de préférence solides dans les conditions de la mesure et peuvent être placés directement sur les unités 20 sans être dispersés dans un fluide, ou bien encore être supportés par un fluide, notamment un liquide.

Dans un premier cas, l'objet ou les objets, solides de préférence, peuvent être dispersés dans un fluide, tel une solution, pour leur manipulation. Dans ce cas, les mesures de courants peuvent être opérées dans cette phase fluidique. Mais, préférentiellement, la phase fluidique est supprimée avant les mesures. Par exemple, le ou les objets sont amenés au contact des unités de mesure en étant porté par une solution (aqueuse notamment). Puis, un séchage assure la suppression de la phase liquide de sorte que les objets subsistent à l'état solide sans environnement liquide pour la phase de mesure.

Dans un autre cas, le ou les objets sont directement posés sur les unités 20 sans recours à une phase fluidique de transport.

Le dispositif de l'invention est un dispositif électronique en ce sens qu'il comprend des parties électriques sur un support qui peut, de préférence, comprendre un substrat par exemple en matériau semi-conducteur, notamment à base de silicium. Il peut donc s'agir d'un dispositif microélectronique.

Dans la suite de la description, on utilise les termes suivants avec définition préférentielle qui y est associée ci-après :
- électrodes : il s'agit d'organes électriquement conducteurs, de forme avantageusement allongée et accessible pour une mise en contact d'une partie d'un objet à caractériser;
- jeu d'électrodes : s'entend d'une série d'au moins une électrode, les électrodes étant reliées à une connexion commune, notamment raccordée à une extrémité de chaque électrode. Une unité de mesure sur laquelle un objet peut être placé, comprend typiquement deux jeux d'électrodes. Celles-ci sont alors interdigitées : chaque jeu d'électrodes forme une structure en peigne, les deux peignes s'enchevêtrant de sorte à alterner, suivant la dimension en largeur des électrodes, les électrodes du premier jeu et du deuxième jeu. Dans un cas limite, l'interdigitation est limitée à la juxtaposition d'une électrode d'un premier jeu (qui ne comporte qu'une électrode) et d'une électrode d'un deuxième jeu (qui ne comporte qu'une électrode).

En référence à la figure 1, un exemple représentant deux unités de mesure 20 est illustré. Les unités 20 sont ici des structures indépendantes formées au niveau d'une première face 4 d'un support 3, par exemple issu d'un process de fabrication sur un substrat en silicium ou un autre matériau semi-conducteur. Chaque unité 20 comprend deux jeux d'électrodes. Les électrodes 11 appartiennent à un premier jeu. Elles sont électriquement conductrices et arrangées de manière parallèle suivant leur direction longitudinale, les électrodes 11 étant avantageusement rectilignes. Elles forment une borne d'un circuit électrique, ouvert en absence d'objet à mesurer, et fermé lorsqu'un objet est placé sur l'unité de mesure. Pour la liaison du jeu d'électrodes 11 au reste du circuit, on peut relier les extrémités proximales des électrodes 11 à une barre de peigne 8 par exemple orientée transversalement aux électrodes 11 et elle-même connectée électriquement à un plot 1 qui peut servir de zone de prise de contact pour une mesure par une partie de mesure du circuit électrique globalement construit. Le plot 1 peut être distant du jeu d'électrodes 11. A ce titre, une ligne d'interconnexion 6, de forme et de longueur variables, peut être présente entre les électrodes 11 et le plot 1.

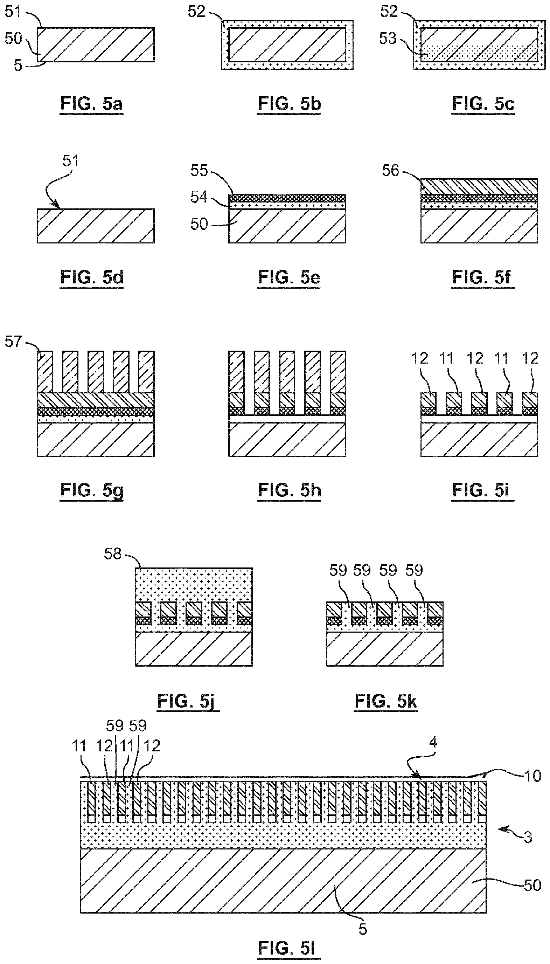

Dans l'illustration de la figure 1, le plot 1 est accessible sur la première face 4 où se situent les électrodes 11. Une configuration alternative consiste à disposer le plot 1 sur une autre face du support 3, par exemple une face opposée (deuxième face 5 décrite plus loin en référence à la figure 5a par exemple) à la première face 4. Dans ce cas, la ligne d'interconnexion 6 peut traverser le support 3, suivant son épaisseur. On peut utiliser des vias traversants, souvent appelés TSV (*Through Silicon Via*) pour les substrats à base de silicium.

Le jeu d'électrodes 11 forme ainsi un premier peigne et leur surface supérieure, c'est-à-dire parallèle à la surface de la première face 4, offre une zone de placement d'objets à caractériser, avec une conduction électrique possible.

L'unité de mesure 20 comprend ensuite un autre jeu d'électrodes 12 complémentaire du jeu d'électrodes 11, en ce sens qu'elles forment une autre borne de raccordement électrique d'un objet placé sur l'unité de mesure 20.

Bien que cela ne soit pas absolument nécessaire, le jeu d'électrodes 12 peut être similaire au jeu d'électrodes 11. Le jeu d'électrodes 12 peut donc lui aussi notamment être relié à un plot 2a, par une ligne d'interconnexion 7a, une barre de peigne 9a servant de reprise électrique commune à une extrémité des électrodes12.

Les électrodes 12 s'organisent de manière interdigitée avec les électrodes 11 de sorte que suivant une direction transversale à la longueur des électrodes 11, 12 (ce qui correspond à la dimension en largeur), les électrodes 11 et 12 sont alternées avec un espace inter-électrodes dans chaque cas. L'interdigitation doit permettre de placer un objet, transversalement à la longueur des électrodes, et de former des contacts entre au moins une électrode 11 et une électrode 12, de sorte à fermer la partie du circuit électrique entre les deux jeux d'électrodes 11, 12.

Avantageusement, au moins un des jeux d'électrodes comprend plus de une électrode 11 ou 12, mais un cas extrême consiste à employer deux jeux de une électrode unique chacun. L'interdigitation est alors une juxtaposition des électrodes 11, 12 suivant une largeur W.

De préférence, pour un jeu d'électrodes 11, 12 donné, la largeur W des électrodes 11, 12 est fixe. Leur longueur l'est aussi avantageusement. La largeur W des électrodes 11, 12 peut être différente entre les deux jeux d'électrodes composant une unité de mesure 20.

Dans le cas de la figure 1, deux unités 20 présentent deux jeux de deux électrodes 11, 12.

L'interdigitation produit un espace entre les électrodes d'une unité de mesure 20. De préférence, cet espace a une même longueur suivant la dimension en largeur W des électrodes. Il est aussi ici appelé distance inter-électrodes G.

On comprend que, lorsqu'un objet est placé sur l'unité de mesure 20, transversalement aux électrodes 11, 12, il entre en contact avec une pluralité d'électrodes 11, 12 si sa dimension est supérieure à la distance G. L'objet peut, de préférence, entrer en contact avec toutes les électrodes 11, 12 de l'unité de mesure 20. Un tel contact, en plusieurs zones de l'objet, suivant la dimension en largeur des électrodes 11, 12, produit une zone de mise en contact discontinue à laquelle une partie de chaque électrode 11, 12 participe. La longueur de cette zone de mise en contact est donc définie par la somme des largeurs des électrodes 11, 12. La largeur de la zone de mise en contact dépend de la dimension de l'objet suivant la longueur des électrodes 11, 12. Cette combinaison de dimension de zones de contact définit une aire de contact globale.

Cette aire est d'autant plus grande que la largeur W des électrodes est importante ou/et que le nombre m d'électrodes des jeux est élevé. Cela est aussi vérifié, même si l'objet n'est pas strictement orienté perpendiculairement aux électrodes.

Lorsqu'un objet est placé sur l'unité de mesure 20, transversalement aux électrodes 11, 12, s'il entre en contact avec seulement une portion des électrodes, la zone de contact sera plus petite que la somme des largeurs des électrodes. Ce cas est particulièrement observé aux extrémités du nano-objet car le début et la fin du nano-objet n'est pas toujours en contact sur toute la largeur de l'électrode mais seulement sur une partie de l'électrode. Il est particulièrement intéressant dans ce cas de procéder à une caractérisation morphologique, par imagerie exemple SEM (microscope à balayage électronique) ou TEM (microscope électronique en transmission) ou encore AFM (microscope à force atomique) afin de déterminer précisément la valeur de la zone de contact. On peut noter que la diminution des valeurs G, W proposée dans l'invention permet de réduire l'incertitude sur ces zones spécifiques.

Lors d'une caractérisation, un type d'objet est généralement à caractériser. Un seul objet peut être suffisant et dans ce cas il sera mis au contact de plusieurs unités 20 ; plusieurs objets peuvent aussi être employés, par exemple deux objets d'un type donné placés sur une zone d'accueil d'une unité différente. Le terme type d'objet signifie que les objets ont au moins une caractéristique identique (notamment matériau et/ou longueur et/ou largeur) si bien qu'ils reflètent une nature commune et une similarité suffisante pour permettre une caractérisation du type d'objet en les caractérisant chacun d'eux. Il n'est pas exclu que les objets d'un type varie quelque peu les uns des autres pour autant. Par exemple, ils peuvent être de longueurs différentes, mais par exemple à chaque fois suffisantes pour coopérer avec toutes ou une majorité des électrodes des unités de mesure.

Une caractérisation peut être opérée avec un contact d'un objet avec au moins deux électrodes de deux unités, l'objet étant par exemple à cheval entre ces unités. Un mesure sera a fortiori possible avec une plus grande coopération entre l'objet et les unités 20 : dans l'illustration de la figure 2, l'objet peut être au contact de toutes les électrodes de l'unité 20 médiane et en contact avec une partie seulement des électrodes d'au moins une des deux autres unités qui l'entourent. L'objet à mesurer est préférentiellement plus long que la série d'unités 20 juxtaposées de sorte à obtenir, comme en figure 2, un contact sur toutes les électrodes.

Toujours en référence à la figure 2, il est possible que les unités présentent toutes des largeurs de zones d'accueil d'objet de même longueur (suivant une direction longue des électrodes perpendiculaire à la largeur W). Dans ce cas il est souhaitable d'aligner les unités de sorte à ce que les zones d'accueil s'enchainent comme en figure 2. Avantageusement, l'écartement entre deux unités de mesure 20 suivant la direction de juxtaposition est inférieure à 10 fois, voire 5 fois (et de préférence 2 fois) la plus valeur de W dans l'ensemble de ces unités 20 juxtaposées.

Lorsqu'un objet est placé sur l'unité de mesure 20, une source de courant permet de faire passer un courant au travers de l'objet. On parle alors de caractérisation électrique d'un nano objet. La caractérisation électrique est définie par exemple par les paramètres tels que le courant, flux d'électron à travers une section d'un nano objet, la conduction électrique ou la résistance. Cette caractérisation électrique peut aussi permettre d'étudier les propriétés de transport et le comportement du nano-objet. En plus de ces différents paramètres, le courant traversant est influencé par un courant parasite dont une partie est due à la résistance des électrodes actives et à la résistance des autres parties intervenant dans le circuit de mesure telle que la résistance de ligne ou celle des pads ou plots. Cette partie de courant parasite peut être déterminée en effectuant une mesure électrique de l'unité en absence de l'objet, par exemple une mesure dite « 4 pointes ». Une autre partie du courant parasite est due à la résistance de contact entre l'électrode et l'objet.

Cette résistance est proportionnelle à l'aire de contact entre l'objet et les électrodes (donc proportionnelle à la longueur de la zone de mise en contact qui dépend du nombre d'électrodes m et de la largeur des électrodes. Rapporté à la longueur de l'objet, il faut aussi tenir compte de la longueur de la zone inter-électrodes qui est la valeur complémentaire de celle de la zone de mise en contact. Ainsi, selon l'invention, on joue sur la valeur d'au moins un paramètre parmi le nombre d'électrodes m, la largeur des électrodes W et la distance qui sépare les électrodes pour faire varier, d'une unité de mesure à l'autre la valeur de la résistance de contact et, portant la valeur du courant parasite. Ensuite, connaissant le courant parasite, on peut déterminer le courant traversant l'objet ou tout objet de même nature.

Par conséquent, l'invention opère une mesure différentielle en employant au moins deux unités de mesure 20 différentes de sorte à présenter des valeurs de courant parasite différentes. Avantageusement, les mêmes paramètres électriques sont employés dans chaque unité de mesure (en particulier valeur du courant) pour cette mesure. De préférence, les électrodes de toutes les unités sont d'un même matériau, ou de matériaux présentant des propriétés de résistivité électrique identiques. De préférence, les électrodes des unités de mesure ne diffèrent exclusivement que par leur nombre et/ou la valeur de G et/ou la valeur de W.

Dans un premier cas, on modifie uniquement la valeur de la largeur des électrodes entre deux unités équivalentes par ailleurs. La variation de courant mesurée correspond à une variation de courant parasite qui peut donc être déterminée.

Dans un autre cas, à même largeur d'électrode W des deux unités, on modifie leur espace inter-électrodes. La différence de courant mesurée est une différence de courant traversant les électrodes.

Une autre possibilité consiste à changer le nombre d'électrodes avec le même effet que dans le premier cas sur la modification de l'aire de contact.

La représentation de la figure 1 montre le cas d'une modification de distance inter-électrodes G entre les unités 20 les autres paramètres étant identiques (même largeur W pour les électrodes 11, 12, 21, 22 et deux électrodes par jeu d'électrodes). Les unités 20 sont ici des structures distinctes.

Des conceptions plus complexes sont possibles telles qu'en figure 2 avec une pluralité d'unités 20 ayant chacune un jeu d'électrodes raccordé à un plot 1 comme les trois unités groupées ont ici des distances inter-électrodes différentes de sorte à mesurer l'objet sur plusieurs distances. Cela est particulièrement adapté aux nano-objets bio inspirés, hétérogènes comme une protéine avec plusieurs fonctions sur sa longueur. Dans le cas de la figure 2, plusieurs unités 20 sont juxtaposées de sorte à s'inscrire en continuité suivant une direction privilégiée qui peut être celle de la largeur des électrodes.

Notamment dans le cas de la figure 2, le positionnement en série des différentes unités de mesure 20 différentes par leurs électrodes (paramètre W et/ou G et/ou nombre d'électrodes) et la mesure différentielle du même nano-objet sur des électrodes de permet de remonter à la contribution de chacun des paramètres participant à la conduction électriques. On peut donc obtenir les véritables propriétés électriques de l'objet ou d'une portion de l'objet car on a retranché les contributions non utiles (telles que contact de surface et/ou résistivité du métal des électrodes etc...).

On notera que dans la figure 2, non seulement les unités 20 sont en enfilade suivant une direction privilégiée de placement d'un objet, mais aussi bénéficie d'une mutualisation d'un raccordement à l'unité de mesure, par des jeux d'électrodes mises en série, les jeux d'électrodes opposés restant raccordés de manière séparée à l'unité de mesure. A ce titre, dans un mode de réalisation, au moins une partie des unités de mesure 20 sont disposées en série, avec respectivement, un jeu d'électrodes relié à un même plot et les autres jeux d'électrodes reliés à des plots distincts.

On notera que l'on peut aussi y faire varier les paramètres W et m.

La compacité de ce dispositif est aussi révélée par la présence de quatre plots 1, 2a, 2b, 2c seulement avec quatre lignes d'interconnexion 6, 7a, 7b, 7c et ce pour six jeux d'électrodes au total.

Les jeux d'électrodes étant rapprochés, les conditions de mesure sont proches (telles que quasi simultanéité, humidité, pression, luminosité) le tout sur un même matériau.

Cette conception peut servir à des protéines de grande longueur de 100nm ou à un objet complexe formé de plusieurs objets élémentaires (tels des nano-objets groupés). Avec un nombre d'électrodes m élevé, on peut caractériser de nombreux tronçons de l'objet et en tirer une valeur moyenne.

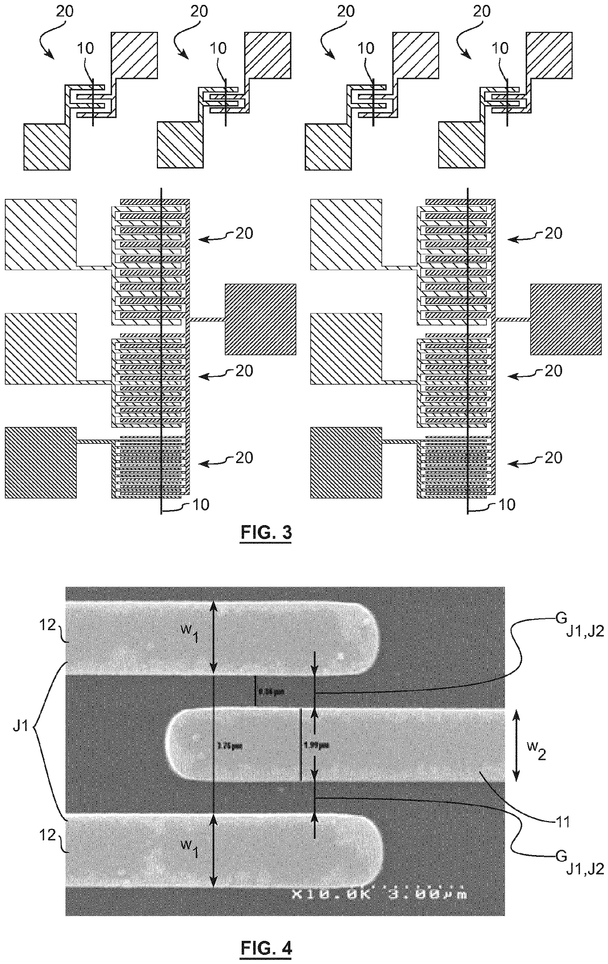

Le nombre d'unités de mesure 20 n'est pas limité selon l'invention. Les multiplier permet d'opérer des mesures en parallèle sur plusieurs objets d'un même type (même matériau, même forme notamment). C'est ce qu'illustre la figure 3 avec dix unités. Quatre d'entre elles sont similaires à celles de la figure 1, pour une mesure d'un premier type d'objet. Six autres sont du mode de la figure 2, à paramètres multiples, appliqué à un autre type d'objet.

La figure 4 illustre une vue au microscope en détail de trois électrodes 11, 12 adjacentes. Suivant cet exemple, les paramètres suivants sont possibles :
- largeur des motifs W des électrodes 11, 12 comprise entre 1 nm à 10µm, mais typiquement dans cet exemple la largeur est de 1.99µm.
- Espace G entre deux électrodes 11, 12 entre 100 nm à 10 µm, mais typiquement entre 500 nm et 1 µm, dans cet exemple la largeur est de 0.86µm.
- Espace entre trois électrodes (entre deux électrodes d'un même jeu) entre 100 nm à 10 µm, mais typiquement dans cet exemple l'espacement est de 3.76 µm.

Cette structure permet ici de caractériser un objet bio inspiré sur des distances de 3.76 µm et 0.86 µm en tenant compte d'une résistance de contact d'électrodes de largeur 1.99 µm en utilisant le design de la figure 1.

Un autre aspect de l'invention est un procédé de fabrication d'un dispositif de mesure à partir d'un substrat 50. Les figures 5a à 5h en montrent un exemple.

La hauteur des électrodes sera ajustée en fonction de l'objet à caractériser et des contraintes de résistance de contact objet/support. La hauteur d'électrode sera comprise de préférence entre 10 nm à plusieurs dizaines de micromètres. Typiquement, la hauteur sera de 200 nm mais peut varier par exemple entre 10 nm et 100 µm.

La figure 5a présente une plaque de matériau semi-conducteur, tel du silicium, pouvant former le substrat 50 de départ du procédé de fabrication. Il comporte une première face 51 par laquelle une unité de mesure va être fabriquée, et une face opposée qui va former la deuxième face 5 du dispositif.

Une étape facultative présentée en figure 5b consiste à former une couche d'oxyde 52, en particulier de silicium, autour du substrat 50, pour protéger les surfaces durant une étape ultérieure, facultative elle aussi, d'implantation ionique. L'oxydation est par exemple issue d'un traitement thermique tel un recuit en atmosphère d'oxygène ou être opérée par dépôt.

L'implantation est schématisée en zone 53 en figure 5c. Elle peut permettre une prise de contact plus efficace par la face arrière 5. Elle peut être effectuée par dopage de type p ou n, par exemple avec des concentrations de dopants entre 10¹⁶ à 10²⁰ at/cm³ suivi d'un recuit d'activation.

La couche d'oxyde 52 issue de la figure 5b peut être retirée, au moins en partie, comme en figure 5d. Une gravure, sèche ou humide, peut convenir. Cette étape est aussi facultative.

Ensuite, le ou les matériaux électriquement conducteurs qui vont servir à former les jeux d'électrodes et toute autre partie conductrice associée, sont déposés après mise en place d'une couche isolante 54 (tel du SiO₂) si le substrat est conducteur. La couche 54 peut être épitaxiée ou déposée notamment par une technique de dépôt classique en phase vapeur, de quelques nanomètres à plusieurs microns typiquement. Du SiH₄ peut aussi être employé.

Pour la partie conductrice, on dépose de préférence une couche d'accroche 55 permettant de réaliser la couche conductrice. On peut utiliser par exemple une couche de titane déposée par la technique de PVD (dépôt physique en phase vapeur) ou électrolyse. L'épaisseur de la couche sera par exemple de l'ordre de quelques nanomètres à plusieurs microns d'épaisseur mais typiquement une couche de 10 nm de titane est suffisante.

En figure 5f, on dépose la couche conductrice 56 par différentes techniques bien connues de l'homme du métier typiquement par PVD, électrolyse ou évaporation. Les matériaux utilisés peuvent être du platine, or mais préférentiellement on utilisera une multi couche dont l'une est composée de Ruthénium. Les épaisseurs seront de l'ordre de quelques nanomètres à quelques microns. Préférentiellement, on déposera deux couches composées d'or de 200 nm puis d'une couche de Ruthénium de 50 nm. Le Ruthénium permet d'obtenir des flancs parfaitement droits et donc de réduire l'espacement entre deux électrodes, du fait de l'anisotropie de sa gravure.

Comme cela est visible en figure 5g, on réalise des motifs de masque 57 afin d'obtenir des électrodes de largeurs très petites (par exemple 100 nm à 10 µm mais typiquement 1 µm). La hauteur des électrodes sera ajustée en fonction du nano objet ou autre objet à caractériser et des contraintes de résistance de contact du l'objet ou du support. La hauteur d'électrode sera comprise entre 10 nm à plusieurs dizaines de micromètres. Typiquement, la hauteur sera de 200 nm.

En figures 5h et 5i, on réalise une gravure de la couche conductrice 55, 56 conformément aux motifs réalisés puis on réalise l'enlèvement de la résine déposée à l'étape de la figure 5g.

Ensuite, on remplit l'espace entre les électrodes par un oxyde. On peut par exemple déposer une couche isolante 58 d'au moins la hauteur des électrodes. Cette couche sera déposée par les techniques conventionnelles telles que PECVD (dépôt chimique en phase vapeur assisté par plasma), LPCVD (dépôt chimique en phase vapeur à basse pression) et autres techniques bien connues de l'homme du métier. Par exemple, on déposera une couche d'oxyde de silicium sur un substrat silicium par PECVD d'une hauteur au moins égale à la hauteur des électrodes. Suivant un exemple, l'épaisseur d'oxyde entre les électrodes sera au moins de 200 nm équivalent à la hauteur des électrodes de 200 nm. Cela est visible en figure 5j.

Dans le cas d'un dépôt sur toute la surface (dépôt conforme), une couche isolante 58 peut être présente sur les électrodes. Une étape de retrait partiel, par polissage tel qu'un *Chemical Mechanical Polishing* (CMP) ou une gravure sèche et/ou humide sera réalisée afin de mettre à nu les électrodes. Par exemple, lors d'un dépôt de 500 nm d'oxyde par PECVD sur un substrat de silicium comprenant une hauteur d'électrode de 200 nm, il sera réalisé un enlèvement de 300 nm d'oxyde. Le résultat est visible en figure 5k.

Comme indiqué précédemment, la couche conductrice 58 peut être en plusieurs sous-couches de matériaux différents.

Le résultat de la figure 5k montre des électrodes affleurant au niveau de la première face 4 du dispositif ainsi construit. De même, des portions non conductrices 59 inter-électrodes sont en affleurement sur la première face 4. Cela offre un contact plan pour l'objet à mesurer, comme cela ressort de la figure 5l.

On notera aussi à cette figure qu'un grand nombre m d'électrodes procure de multiples tronçons de mesure de l'objet.

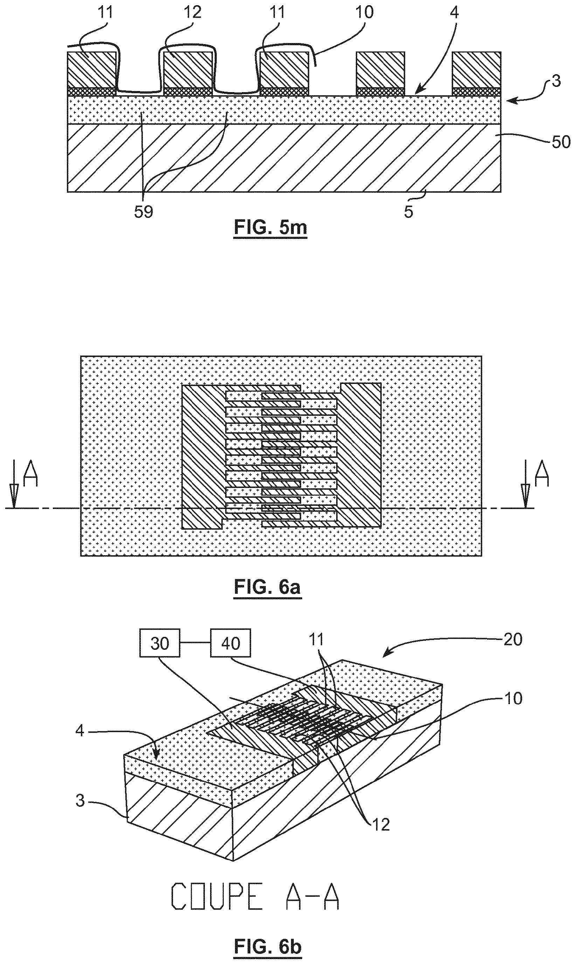

Dans une variante illustrée en figure 5m, les portions non conductrices 59 sont à un niveau de hauteur inférieur à celui des électrodes 11, 12 si bien que l'objet 10, particulièrement s'il est filiforme tel une protéine filiforme, est déformé suivant l'épaisseur du substrat, et non pas dans le plan de la face supérieure des électrodes 11, 12. Ce cas est généralement moins favorable que celui de la figure 5l. Le mode de réalisation en affleurement des électrodes 11, 12 relativement aux portions non conductrices, de sorte que l'ensemble est dans le plan de la face du support 3 est donc préféré. Cette caractéristique de formation des électrodes 11, 12 en affleurement peut d'ailleurs être mise en oeuvre indépendamment des autres aspects de l'invention, par exemple ceux liés à la mesure différentielle avec plusieurs unités de mesure. Ainsi, l'invention concerne aussi un dispositif de mesure d'une caractéristique électrique d'un objet comprenant une unité de mesure au moins, avec deux jeux d'électrodes dont la surface supérieure est en affleurement relativement à la partie de la face du support qui entoure les électrodes, dont des portions conductrices inter-électrodes.

Ce principe d'affleurement est aussi illustré aux figures 6a et 6b où l'isolant entoure les électrodes 11, 12. La figure 6b montre aussi schématiquement le circuit de mesure.

Le circuit de mesure comprend typiquement une source d'alimentation électrique 30 et au moins un système de mesure 40 de grandeurs électriques, en particulier des courants, ces éléments étant reliés de façon fixe ou non aux électrodes, de préférence via des plots 1, 2a, 2b, 2c (non visibles en figure 6b). On peut ainsi, avec une mesure du courant aux bornes des plots 1, 2a, 2b, 2c, évaluer la contribution électrique de l'objet aux courants mesurés, en tenant avantageusement compte des courants parasites.

Les courants sont souvent faibles pour des nano-objets, par exemple entre 5-10⁻⁹ A et 5.10⁻⁸ A pour une protéine amyloïde.

Suivant un procédé de mesure de l'invention, on place au moins un objet sur une unité de mesure en s'arrangeant pour qu'il ait une orientation transversale à la longueur des électrodes. Le placement peut avoir lieu par dépôt de gouttes de solution dans laquelle le ou les objets à caractériser sont présents. Le dispositif lui-même peut aussi être plongé, au moins par une face où se trouvent des unités de mesure, dans une solution contenant les objets.

La présence de multiples unités de mesure permet d'accroître les probabilités d'un bon positionnement des objets sur les unités. Lorsque les objets placés sur les unités sont identiques (en particulier même matériau et mêmes dimensions) des mesures simultanées sont possibles.

### REFERENCES

- 1.: Plot
- 2a, 2b, 2c.: Plot
- 3.: Support
- 4.: Première face
- 5.: Deuxième face
- 6.: Ligne d'interconnexion
- 7a, 7b, 7c.: Ligne d'interconnexion
- 8.: Barre de peigne
- 9a, 9b, 9c.: Barre de peigne
- 10.: Objet
- 11.: Electrode
- 12.: Electrode
- 20.: Unité de mesure
- 21.: Electrode
- 22.: Electrode
- 30.: Source
- 40.: Circuit de mesure
- 31.: Electrode
- 32.: Electrode
- 50.: Substrat
- 51.: Première face du substrat
- 52.: Couche d'oxyde
- 53.: Zone d'implantation
- 54.: Couche isolante
- 55.: Couche d'accroche conductrice
- 56.: Deuxième couche conductrice
- 57.: Masque
- 58.: Couche isolante
- 59.: Portion non conductrice
- W.: Direction en largeur
- G.: Distance inter-électrodes
- Z :: Zone d'accueil du nano objet

## Revendications

1. Dispositif électronique de mesure d'au moins une caractéristique électrique d'un type donné d'objet (10), comportant :
- un support (3) pourvu d'au moins deux unités de mesure (20) comportant chacune au moins deux jeux d'électrodes Ji avec i entier supérieure ou égal à 2, chaque jeu (Ji) d'électrodes comprenant un nombre mi d'électrodes supérieur ou égal à un, les électrodes d'un jeu Ji d'électrodes ayant une dimension en largeur (WJi), les électrodes des jeux d'électrodes d'une même unité de mesure (20) étant interdigitées de sorte que chaque électrode de l'un des jeux d'électrodes Ji de ladite unité de mesure (20) soit espacée d'une distance inter-électrodes (G(JiJi+1)) d'une électrode, de l'autre des jeux d'électrodes Ji+1 de ladite unité de mesure (20), qui lui est adjacente; les électrodes des jeux d'électrodes d'une unité de mesure formant pour ladite unité de mesure (20), une zone d'accueil (ZJ1J2) d'au moins un objet (10) du type donné définissant, dans cette zone d'accueil, une zone de mise en contact discontinue avec les électrodes de l'unité de mesure apte à être mise en contact avec un objet (10) du type donné, ladite zone de mise en contact ayant une longueur déterminée suivant la dimension en largeur (W) des électrodes correspondant à la somme des largeurs (WJi) des électrodes, et chaque zone d'accueil comportant en outre une zone inter-électrodes discontinue ayant une longueur prédéterminée suivant la dimension en largeur (W) des électrodes de ladite unité de mesure (20) ;
- un circuit de mesure raccordé aux jeux d'électrodes de chaque unité de mesure pour déterminer, pour chaque unité de mesure, une valeur de courant traversant l'objet;
dispositif dans lequel au moins deux unités de mesure (20) diffèrent entre elles par la longueur de leurs zones de mise en contact et/ou la longueur de leurs zones inter-électrodes, le circuit de mesure étant configuré pour déterminer une différence des valeurs de courant des au moins deux unités de mesure (20) et pour déterminer une valeur différentielle de résistance électrique de contact entre un objet du type donné et les électrodes de chacune des unités de mesure (20).

2. Dispositif selon la revendication précédente, dans lequel, pour au moins une unité de mesure (20), au moins un jeu d'électrodes (Ji) comprend un nombre mi d'électrodes supérieur ou égale à 2.

3. Dispositif selon l'une des revendications précédentes, dans lequel chaque unité de mesure (20) présente une dimension en largeur inférieure ou égale à une dimension en longueur du type donné d'objet.

4. Dispositif selon l'une des revendications précédentes, comprenant au moins deux unités de mesure juxtaposées suivant la dimension en largeur (W).

5. Dispositif selon l'une des revendications précédentes, dans lequel, dans chaque jeu d'électrodes de chaque unité de mesure (20), la dimension en largeur (W) des électrodes est identique et dans lequel la longueur de la zone de mise en contact d'au moins deux unités de mesure diffère par la largeur (W) des électrodes d'au moins un jeu d'électrodes de chacune desdites au moins deux unités de mesure (20).

6. Dispositif selon l'une des revendications précédentes, dans lequel la longueur de la zone de mise en contact d'au moins deux unités de mesure (20) diffère par le nombre (m) d'électrodes de leurs jeux d'électrodes.

7. Dispositif selon l'une des revendications précédentes, dans lequel, dans chaque jeu d'électrodes de chaque unité de mesure (20), la distance inter-électrodes est identique et dans lequel les longueurs des zones inter-électrodes d'au moins deux unités de mesure (20) diffèrent par leurs distances inter-électrodes.

8. Dispositif selon l'une des revendications précédentes, dans lequel le circuit de mesure comporte des plots (1, 2a, 2b, 2c), les jeux d'électrodes d'une même unité de mesure (20) étant reliés à des plots (1, 2a, 2b, 2c) distincts et dans lequel dans le circuit de de mesure comporte au moins un plot relié (1) à plusieurs jeux d'électrodes appartenant chacune à une unité de mesure (20) différente.

9. Dispositif selon la revendication précédente, dans lequel au moins un plot est situé sur une face du support (3) différant d'une face (4) sur laquelle est située l'unité de mesure (20) à laquelle ledit au moins un plot (1, 2a, 2b, 2c) est relié, et en ce que le circuit de mesures comprend une partie de raccordement entre ledit au moins un plot (1, 2a, 2b, 2c) de l'unité de mesure (20) avantageusement traversant le support.

10. Dispositif selon l'une des revendications précédentes, dans lequel les électrodes des jeux d'électrodes d'au moins une unité de mesure sont affleurantes sur une face du support (3) et dans lequel les électrodes des jeux d'électrodes de ladite au moins une unité de mesure (20) sont espacées par des portions non conductrices affleurant sur la face (4) du support (3).

11. Dispositif selon l'une des revendications précédentes, dans lequel la dimension en largeur (W) des électrodes est inférieure à 1 µm et/ou la distance inter-électrodes est comprise entre 500 nm et 1 µm.

12. Dispositif selon l'une des revendications précédentes, dans lequel le support (3) comporte une première face pourvue d'au moins une unité de mesure (20) et une deuxième face différente de la première face, pourvue d'au moins une unité de mesure (20).

13. Système comprenant au moins un objet d'un type donné et un dispositif selon l'une des revendications précédentes, au moins deux unités de mesure (20) recevant un objet sur leur zone d'accueil respective.

14. Procédé de mesure d'au moins une caractéristique électrique d'un objet, comprenant :
- l'utilisation d'un dispositif selon l'une des revendications 1 à 12 ;
- une étape de placement d'un objet (10) d'un type donné sur la zone d'accueil d'unité de mesure (20) et une étape de mesure du courant circulant entre les jeux d'électrodes de ladite unité (20);
- une étape de placement de l'objet (10) ou d'un objet du type donné sur la zone d'accueil d'une autre unité de mesure (20) et une étape de mesure du courant circulant entre les jeux d'électrodes de ladite autre unité;
- une détermination d'une différence des valeurs de courant des unités de mesure (20) et une détermination d'une valeur différentielle de résistance électrique de contact entre un objet du type donné et les électrodes de chacune des unités de mesure (20) ;
- une évaluation du courant traversant l'objet à partir des mesures de courant réalisées aux étapes précédentes.

15. Procédé selon la revendication précédente, dans lequel le type d'objet est à l'état solide.

16. Procédé selon l'une des deux revendications précédentes, dans lequel on utilise un dispositif qui comprend au moins deux unités de mesure juxtaposées suivant la dimension en largeur (W) et on place un objet du type donné simultanément sur les au moins deux unités de mesure juxtaposées.

## Patentansprüche

1. Elektronische Vorrichtung zum Messen mindestens eines elektrischen Parameters eines bestimmten Typs eines Gegenstands (10), umfassend:
- einen Träger (3), der mit mindestens zwei Messeinheiten (20) versehen ist, wobei jede mindestens zwei Elektrodensätze Ji umfasst mit i als ganzer Zahl größer oder gleich 2, jeder Elektrodensatz (Ji) eine Anzahl mi von Elektroden umfasst, die größer oder gleich eins ist, die Elektroden eines Elektrodensatzes Ji eine Breitenabmessung (WJi) aufweisen, die Elektroden der Elektrodensätze einer selben Messeinheit (20) so ineinandergreifen, dass jede Elektrode des einen der Elektrodensätze Ji der Messeinheit (20) mit einem interelektrodischen Abstand (G(JiJi+1)) von einer Elektrode des anderen der Elektrodensätze Ji+1 der Messeinheit (20) angeordnet ist, die an diese angrenzt; wobei die Elektroden der Elektrodensätze einer Messeinheit für diese Messeinheit (20) eine Aufnahmezone (ZJ1J2) für mindestens einen Gegenstand (10) des bestimmten Typs bilden, in dieser Aufnahmezone eine diskontinuierliche Kontaktierzone mit den Elektroden der Messeinheit definiert wird, die geeignet ist, mit einem Gegenstand (10) des bestimmten Typs in Kontakt gebracht zu werden, die Kontaktierzone eine Länge aufweist, die gemäß der Breitenabmessung (W) der Elektroden entsprechend der Summe der Breiten (WJi) der Elektroden bestimmt wird, und jede Aufnahmezone ferner eine diskontinuierliche interelektrodische Zone umfasst, die eine Länge aufweist, die gemäß der Breitenabmessung (W) der Elektroden der Messeinheit (20) vorgegeben wird;
- eine Messschaltung, die mit den Elektrodensätzen jeder Messeinheit verbunden ist, um für jede Messeinheit einen durch den Gegenstand fließenden Stromwert zu bestimmen;
wobei sich in der Vorrichtung mindestens zwei Messeinheiten (20) untereinander hinsichtlich der Länge ihrer Kontaktierzonen und/oder der Länge ihrer interelektrodischen Zonen unterscheiden, wobei die Messschaltung dazu ausgelegt ist, einen Unterschied der Stromwerte der mindestens zwei Messeinheiten (20) zu bestimmen und einen Differenzwert des elektrischen Kontaktwiderstands zwischen einem Gegenstand des bestimmten Typs und den Elektroden einer jeden Messeinheit (20) zu bestimmen.

2. Vorrichtung nach dem vorangehenden Anspruch, bei der für zumindest eine Messeinheit (20) zumindest ein Elektrodensatz (Ji) eine Anzahl mi von Elektroden umfasst, die größer oder gleich 2 ist.

3. Vorrichtung nach einem der vorangehenden Ansprüche, bei der jede Messeinheit (20) eine Breitenabmessung aufweist, die kleiner oder gleich der Längenabmessung des bestimmten Gegenstandstyps ist.

4. Vorrichtung nach einem der vorangehenden Ansprüche, umfassend mindestens zwei Messeinheiten, die gemäß der Breitenabmessung (W) nebeneinander angeordnet sind.

5. Vorrichtung nach einem der vorangehenden Ansprüche, bei der in jedem Elektrodensatz einer jeden Messeinheit (20) die Breitenabmessung (W) der Elektroden identisch ist und bei der sich die Länge der Kontaktierzone von mindestens zwei Messeinheiten durch die Breite (W) der Elektroden von mindestens einem Elektrodensatz einer jeden der mindestens zwei Messeinheiten (20) unterscheidet.

6. Vorrichtung nach einem der vorangehenden Ansprüche, bei der sich die Länge der Kontaktierzone von mindestens zwei Messeinheiten (20) durch die Anzahl (m) der Elektroden ihrer Elektrodensätze unterscheidet.

7. Vorrichtung nach einem der vorangehenden Ansprüche, bei der in jedem Elektrodensatz einer jeden Messeinheit (20) der interelektrodische Abstand identisch ist und bei der sich die Längen der interelektrodischen Zonen von mindestens zwei Messeinheiten (20) durch ihren interelektrodischen Abstand unterscheiden.

8. Vorrichtung nach einem der vorangehenden Ansprüche, bei der die Messschaltung Kontaktstücke (1, 2a, 2b, 2c) aufweist, wobei die Elektrodensätze einer selben Messeinheit (20) mit verschiedenen Kontaktstücken (1, 2a, 2b, 2c) verbunden sind und bei der in der Messschaltung mindestens ein Kontaktstück (1) vorhanden ist, das mit mehreren Elektrodensätzen verbunden ist, die jeweils zu einer unterschiedlichen Messeinheit (20) gehören.

9. Vorrichtung nach dem vorangehenden Anspruch, bei der mindestens ein Kontaktstück auf einer Seite des Trägers (3) vorgesehen ist, die sich von einer Seite (4) unterscheidet, auf der die Messeinheit (20) vorgesehen ist, mit der das mindestens eine Kontaktstück (1, 2a, 2b, 2c) verbunden ist, und bei der die Messschaltung einen Verbindungsabschnitt zu dem mindestens einen Kontaktstück (1, 2a, 2b, 2c) der Messeinheit (20) umfasst, vorzugsweise den Träger durchquerend.

10. Vorrichtung nach einem der vorangehenden Ansprüche, bei der die Elektroden der Elektrodensätze von mindestens einer Messeinheit bündig auf einer Seite des Trägers (3) sind und bei der die Elektroden der Elektrodensätze der mindestens einen Messeinheit (20) durch nichtleitende Abschnitte beabstandet sind, die bündig auf der Seite (4) des Trägers (3) sind.

11. Vorrichtung nach einem der vorangehenden Ansprüche, bei der die Breitenabmessung (W) der Elektroden geringer als 1 µm ist und/oder der interelektrodische Abstand zwischen 500 nm und 1 µm beträgt.

12. Vorrichtung nach einem der vorangehenden Ansprüche, bei der der Träger (3) eine erste Seite, die mit mindestens einer Messeinheit (20) versehen ist, und eine von der ersten Seite verschiedene zweite Seite aufweist, die mit mindestens einer Messeinheit (20) versehen ist.

13. System, umfassend mindestens einen Gegenstand eines bestimmten Typs und eine Vorrichtung nach einem der vorangehenden Ansprüche, wobei mindestens zwei Messeinheiten (20) in ihren jeweiligen Aufnahmezonen einen Gegenstand empfangen.

14. Verfahren zum Messen mindestens eines elektrischen Parameters eines Gegenstands, umfassend:
- die Verwendung einer Vorrichtung nach einem der Ansprüche 1 bis 12;
- einen Schritt des Anordnens eines Gegenstands (10) eines bestimmten Typs auf der Aufnahmezone einer Messeinheit (20) und einen Schritt des Messens des zwischen den Elektrodensätzen der Einheit (20) fließenden Stroms;
- einen Schritt des Anordnens des Gegenstands (10) oder eines Gegenstands vom bestimmten Typ auf der Aufnahmezone einer anderen Messeinheit (20) und einen Schritt des Messens des zwischen den Elektrodensätzen der anderen Einheit (20) fließenden Stroms;
- eine Bestimmung einer Differenz der Stromwerte der Messeinheiten (20) und eine Bestimmung eines Differenzwertes des elektrischen Kontaktwiderstands zwischen einem Gegenstand vom bestimmten Typ und den Elektroden einer jeden der Messeinheiten (20);
- eine Bewertung des den Gegenstand durchfließenden Stroms anhand der in den vorangehenden Schritten ausgeführten Messungen des Stroms.

15. Verfahren nach dem vorangehenden Anspruch, bei dem der Typ des Gegenstands in einem festen Zustand besteht.

16. Verfahren nach einem der zwei vorangehenden Ansprüche, bei dem eine Vorrichtung verwendet wird, die mindestens zwei Messeinheiten umfasst, die gemäß der Breitenabmessung (W) nebeneinander angeordnet sind, und ein Gegenstand vom bestimmten Typ gleichzeitig auf den mindestens zwei nebeneinander angeordneten Messeinheiten angeordnet wird.

## Claims

1. Electronic device for measuring at least one electrical characteristic of a given type of object (10), comprising:
- a supporting base (3) provided with at least two measuring units (20) each comprising at least two sets of electrodes Ji with i integer greater than or equal to 2, each set (Ji) of electrodes comprising a number mi of electrodes greater than or equal to one, the electrodes of a set Ji of electrodes having a dimension in width (WJi), the electrodes of the sets of electrodes of the same measuring unit (20) being interdigitated such that each electrode of one of the sets of electrodes Ji of said measuring unit (20) is spaced by an inter-electrode distance (G(JiJi+1)) from an electrode, of the other of the sets of electrodes Ji+1 of said measuring unit (20), which is adjacent thereto; the electrodes of the sets of electrodes of a measuring unit forming for said measuring unit (20), a zone (ZJ1J2) for receiving at least one object (10) of the given type defining, in this receiving zone, a discontinuous contacting zone with the electrodes of the measuring unit able to be in contact with an object (10) of the given type, said contacting zone having a predetermined length according to the dimension in width (W) of the electrodes corresponding to the sum of the widths (WJi) of the electrodes, and each receiving zone further comprising a discontinuous inter-electrode zone having a predetermined length according to the dimension in width (W) of the electrodes of said measuring unit (20);
- a measurement circuit connected to the sets of electrodes of each measuring unit to determine the current passing through the object;
device wherein at least two measuring units (20) differ from each other by the length of the contacting zones thereof and/or the length of the inter-electrode zones thereof, the measurement circuit being configured to determine a difference in the current values of the at least two measuring units (20) and to determine a differential electrical contact resistance value between an object of the given type and the electrodes of each of the measuring units (20).

2. Device according to the preceding claim, wherein, for at least one measuring unit (20), at least one set of electrodes (Ji) comprises a number mi of electrodes greater than or equal to 2.

3. Device according to any one of the preceding claims, wherein each measuring unit (20) has a dimension in width less than or equal to a dimension in length of the given type of object.

4. Device according to any one of the preceding claims, comprising at least two measuring units juxtaposed along the dimension in width (W).

5. Device according to any one of the preceding claims, wherein, in each set of electrodes of each measuring unit (20), the dimension in width (W) of the electrodes is identical and wherein the length of the contacting zone of at least two measuring units differs by the width (W) of the electrodes of at least one set of electrodes of said at least two measuring units (20).

6. Device according to any one of the preceding claims, wherein the length of the contacting zone of at least two measuring units (20) differs by the number (m) of electrodes of the sets of electrodes thereof.

7. Device according to any one of the preceding claims, wherein, in each set of electrodes of each measuring unit (20), the inter-electrode distance is identical and wherein the lengths of the inter-electrode zones of at least two measuring units (20) differ by the inter-electrode distances thereof.

8. Device according to any one of the preceding claims, wherein the measurement circuit comprises contact elements (1, 2a, 2b, 2c), the sets of electrodes of the same measuring unit (20) being connected to separate contact elements (1, 2a, 2b, 2c) and wherein the measurement circuit comprises at least one contact element (1) connected to a plurality of sets of electrodes each belonging to a different measuring unit (20).

9. Device according to the preceding claim, wherein at least one contact element is situated on a supporting base face (3) differing from a face (4) whereon the measuring unit (20) is situated to which said at least one contact element (1, 2a, 2b, 2c) is connected, and in that the measurement circuit comprises a connection part between said at least one contact element (1, 2a, 2b, 2c) of the measuring unit (20) advantageously passing through the supporting base.

10. Device according to any one of the preceding claims, wherein the electrodes of the sets of electrodes of said at least one measuring unit are flush on one face of the supporting base (3) and wherein the electrodes of the sets of electrodes of said at least one measuring unit (20) are spaced by flush non-conductive portions on the face (4) of the supporting base (3).

11. Device according to any one of the preceding claims, wherein the dimension in width (W) of the electrodes is less than 1 µm and/or the inter-electrode distance is between 500 nm and 1 µm.

12. Device according to any one of the preceding claims, wherein the supporting base (3) comprises a first face provided with at least one measuring unit (20) and a second face different to the first face, provided with at least one measuring unit (20).

13. System comprising at least one object of a given type and a device according to any one of the preceding claims, at least two measuring units (20) receiving an object on the respective receiving zone thereof.

14. Method for measuring at least one electrical characteristic of an object, comprising:
- the use of a device according to any one of claims 1 to 13;
- a step for placing an object (10) of a given type of the zone for receiving a measuring unit (20) and a step for measuring the current circulating between the sets of electrodes of said unit (20);
- a step for placing the object (10) or an object of the given type on the zone for receiving a further measuring unit (20) and a step for measuring the current circulating between the sets of electrodes of said further unit;
- a determination of a difference in the current values of the measuring units (20) and a determination of a differential contact resistance value between an object of the given type and the electrodes of each of the measuring units (20);
- an evaluation of the current passing through the object on the basis of the current measurements made in the preceding steps.

15. Method according to the preceding claim, wherein the object type is in the solid state.

16. Method according to any one of the two preceding claims, wherein a device is used comprising at least two juxtaposed measuring units along the dimension in width (W) and an object of the given type is placed simultaneously on the at least two juxtaposed measuring units.
